# EUROPÄISCHE PATENTSCHRIFT

(11) **EP 0 634 909 B1**
(45) Veröffentlichungstag und Bekanntmachung des Hinweises auf die Patenterteilung: **19.08.1998**
(21) Anmeldenummer: 93907786.3
(22) Anmeldetag: 02.04.1993
(51) Int. Cl.: A61B 17/36, A61N 5/06

(54) **ARBEITSSCHAFT FÜR DIE PHOTO-THERMO-THERAPIE**
WORKING SHAFT FOR PHOTO-THERMAL THERAPY
TIGE D'APPLICATION DE PHOTO-THERMOTHERAPIE

(30) Priorität: 06.04.1992 DE 4211526; 30.10.1992 DE 4237286
(43) Veröffentlichungstag der Anmeldung: 25.01.1995
(73) Patentinhaber: CeramOptec GmbH, 53121 Bonn (DE); HÜTTINGER MEDIZINTECHNIK GmbH & CO. KG, D-79224 Umkirch (DE)
(72) Erfinder: BERLIEN, Hans-Peter, D-1000 Berlin 33 (DE); MÜLLER, Gerhard, D-1000 Berlin 38 (DE); BEUTHAN, Jürgen, D-1000 Berlin 41 (DE); PHILIPP, Carsten, D-1000 Berlin 46 (DE)
(86) Internationale Anmeldenummer: DE9300321
(87) Internationale Veröffentlichungsnummer: WO9319680

(56) Entgegenhaltungen:
- EP-A- 246 127
- EP-A- 255 974
- DE-A- 3 532 604
- DE-A- 3 941 705

## Beschreibung

Die Erfindung betrifft einen Arbeitsschaft für die Photo-Thermo-Therapie der im Oberbegriff des Anspruchs 1 angegebenen Art.

Aus der DE-C-40 41 234 ist es bekannt, optische Strahlung durch Lichtleitfasern und Kathetersysteme in das Körperinnere zu übertragen, wobei die Lichtleitfasern jeweils distal mit geeignetern Streukörpern versehen sind.

Aus der DE-A-3 941 705 ist bekannt, daß Hohlräume von kugelähnlicher Gestalt mit Hilfe einer Vorrichtung bestrahlt werden können, die aus einem Katheter besteht in dessen Katheterkanal sich eine verschiebbare Lichtleitfaser befindet und dessen dicht aufsitzende verformbare Hülle durch einen getrennten Kanal mit einer Flüssigkeit befüllbar ist. Die Hauptaufgabe der Erfindung besteht in der Schaffung einer Vorrichtung, die eine möglicht kugelförmige Abstrahlcharakteristik insbesondere auf die rückwärtige Hemisphäre gewährleistet und durch enge Zugangsöffnungen in Hohlorgane eingeführt werden kann. Im speziellen besitzt die optische Faser ein konisches Ende, welches von einem Streumedium umgeben ist. In einer Version ist die Flüssigkeit in der Hülle das Streumedium.

Ferner ist aus der DE-C-35 12 018 ein Arbeitsschaft bekannt, bei dem ein Fluid zur Kühlung und als Lichtleiter dient.

In EP-A-0 246 127 wird eine transparente Flüssigkeit als Kühlmedium in einem kardio-vasculären Katheter genutzt, der eine als Linse ausgebildete Endkappe besitzt. Im Innern des distalen Endes des Katheters befindet sich eine Laserfaser, die durch diese Kühlflüssigkeit umspült wird.

Bei dem Versuch, derartige Systeme auch im Rahmen der endoskopischen Chirurgie bzw. interventionell zu nutzen, ergeben sich jedoch eine Reihe von gravierenden Problemen. Insbesondere treten in unmittelbarerer Nähe des distalen Endes des Arbeitsschaftes unzulässige Erwärmungen auf, die eine unzulässige Beeinträchtigung bilden können.
Ferner ist die Abstrahlrichtung nicht steuerbar und somit die Wirkung nicht gleichzeitig visuell beobachtbar bzw. generell modifizierbar.
Der Erfindung liegt die Aufgabe zugrunde, bei einem Arbeitsschaft der eingangs genannten Gattung die Möglichkeit zu schaffen, ein Lichtbündel im Inneren des Körpers auf besonders einfache Weise derart zu applizieren, daß die Erwärmung im Nahbereich unzulässige Werte nicht überschreitet.
Insbesondere soll die thermische Behandlung in Intensität und Richtung in weiten Grenzen von außerhalb des Körpers beeinflußbar sein.

Diese Aufgabe wird mit den kennzeichnenden Merkmalen des Anspruchs 1 gelöst.

Der Erfindung liegt die Erkenntnis zugrunde, daß die in einfacher Weise mittels der heute marktgängigen Endoskopie- und Kathetersystemen im Körperinneren auslös- und steuerbaren photochemischen und photothermischen Reaktionen in ihren Einsatzmöglichkeiten noch wesentlich verbessert werden können, wenn zwischen dem Austrittsbereich der Strahlung aus einem Lichtwellenleiter und einem für die Strahlung transparenten Dom am distalen Ende des Arbeitsschaftes ein Fluid vorgesehen ist, welches einerseits die Funktion der Streuung der Strahlung im Sinne einer Vergleichmäßigung übernimmt, um eine zu starke Konzentration im Auftreffbereich zu unterbinden, und andererseits eine lokale Wärmeabfuhr in der unmittelbaren Nachbarschaft des distalen Endes ermöglicht.

Dabei wurde weiterhin erkannt, daß durch die nichtlineare Abnahme der durch Strahlung erzeugten Erwärmung mit zunehmender Entfernung im Nahbereich des distalen Endes des Arbeitsschaftes die Abfuhr überschüssiger Wärme, die zu sogenannten "Hot spots" und einer lokalen Karbonisierung des Gewebes führen kann, durch Wärmeleitung allein nicht ausreichend ist. Erst durch die Möglichkeit, den Wärmetransport durch Umpumpen des Kühlmediums zu verbessern, läßt sich hier Abhilfe schaffen. Durch ein verdampfbares Fluid wird dabei die Kühlwirkung gegebenenfalls zusätzlich gesteigert.

Die Abmessungen des Querschnitts des Arbeitsschaftes sind normalerweise aus Gründen der Einführbarkeit begrenzt, so daß damit auch das Zentrum der Strahlung auf einen verhältnismäßig kleinen Bereich konzentriert sein muß. Bei einer anderen vorteilhaften Weiterbildung der Erfindung ist der Endbereich des Schafts jedoch expansibel, so daß auch hierdurch eine Vergrößerung der Oberfläche des der Kühlung zugänglichen Austrittsbereichs erzielbar ist.

Auf diese Weise kann die auftretende Erwärmung im wesentlichen unmittelbar im Bereich der Entstehung abgeführt werden. Damit liegt am Applikationsort eine ungerichtet diffuse, aber vergleichmäßigte Strahlung vor, die durch im Bereich des distalen Endes gelegene strahlrichtungsbestimmende Elemente - wie sie beispielsweise durch eine vorbestimmte Ausgestaltung der Austritts- oder Reflexionsflächen erfolgen kann, willkürlich in einer oder mehreren vorbestimmten Raumrichtung konzentriert oder in diese abgelenkt werden kann. Insbesondere kann dabei in vorwählbaren Raumwinkeln bzw. -richtungen und isotrop optische Strahlung im Wellenlängenbereich von 400 nm bis 1400 nm übertragen werden. Zur Ausführung der Erfindung kommen die unterschiedlichsten einschägigen physikalischen Streuungsmechanismen in Flüssigkeiten in Betracht.

Es kann dabei insbesondere eine Vorrichtung in der Form eines starren oder flexiblen Arbeitsschaftes zur Photo-Thermotherapie geschaffen werden, bei der eine Öl-Wasser-Emulsion als flüssiges Streumedium in den Abstrahlbereich einer optischen Faser gespült wird, um so die aus der optischen Faser austretende Laserstrahlung isotrop zu streuen. Dabei ist ebenfalls vorgesehen, daß dieses flüssige Streumedium vorgekühlt wird, um an der Grenzfläche zwischen optischem Arbeitsschaft und zu bestrahlendem Gewebe entstehende Temperaturerhöhungen zu kompensieren. Diese Vorgehensweise ist für eine Vielzahl von Anwendungen äußerst vorteilhaft.

Bei einer bevorzugten Weiterbildung der Erfindung wird über den Arbeitskanal eines Endoskops wird ein zweiter Optikschaft vorgeschoben, der am distalen Ende einen flüssigkeitsdichten für den jeweiligen Applikationswellenlängenbereich aber optisch durchlässigen Dom spezieller Formgebung aufweist, wobei die Formgebung in Kombination mit den Streueigenschaften des Dommaterials derart gewählt wird, daß vorherbestimmbare Volumenelemente hinreichend isotrop mit optischer Strahlung beaufschlagt werden können. Dabei hat sich überraschenderweise gezeigt, daß opalisierende Materialien sowohl in Kristall- als auch in Glasform in besonderer Weise zur Lichtstreuung geeignet sind, inbesondere bei der Verwendung eines ND:YAG- oder Dioden-Lasers.

Dabei wird bevorzugt die Strahlung des Lasers durch einen optischen Lichtwellenleiter an eine proximale optische Schnittstelle des Endoskops und innerhalb des Endoskops mit geeigneter Relaisoptik zum distalen Ende geführt. Bevorzugt ist die Relaisoptik so ausgelegt, daß der distal angebrachte Streulichtdom möglichst homogen ausgeleuchtet wird. Je nach Größe des Endoskopssystems wird in Weiterführung des Erfindungsgedankens die energieübertragende Lichleitfaser auch innerhalb des Endoskopschaftes weitergeführt bis zum distal angebrachten Streulichtdom. In diesem Falle ist jedoch erfindungsgemäß die numerische Apertur der Lichtleitfaser dem gewünschten Raumwinkel anzupassen.

In Weiterführung des Erfindungsgedankens wird das gesamte in den Arbeitskanal eines Endoskops eingeführte Streulichtrohr mit einer Flüssigkeit gespült, um eine Aufwärmung des Applikators selbst durch vagabundierendes Streulicht der Laserstrahlung zu vermeiden.

In einer anderen bevorzugten Ausführungsform wird dabei als Kühlmedium wiederum eine lichtstreuende Flüssigkeit verwendet, wie beispielsweise eine Öl-Wasser-Emulsion, die in dem bevorzugten Ausführungsbeispiel aus Silikonöl und Wasser (ggf. versetzt mit geeigneten Detergentien) in einem Ultraschallbad hergestellt wird, um sodann über eine Umwälzpumpe durch den Endoskopschaft koaxial geführt wird, wobei durch die Frequenz und Intensität des Ultraschallbades die Streueigenschaften der Öl-Wasser-Emulsion in weiten Bereich eingestellt werden können, um damit die Abstrahlcharakteristik am distalen Ende des optischen Streurohres in voherstimmbarer Weise verändert werden kann. In einem weiteren bevorzugten Ausführungsbeispiel wird anstelle des starren optischen Streurohres ein flexibler Katheterschlauch verwendet, der am distalen Ende anstelle des optischen Streudoms eine expandierbare Verschlußkappe trägt, die für die benutzte Wirkstrahlung transparent ist und nicht absorbiert.

Dieser so abgeschlossene Katheterschlauch wird dann ebenfalls wieder (wie am oben ausgeführten Beispiel des starren Streurohres) mit einer biokompatiblen Streuflüssigkeit, beispielsweise einer Fett/Öl-Wasser-Emulsion - wie einer Infusionslösung - durchströmt. Die Laserstrahlung wird im Inneren des Katheters über eine reguläre Lichtleitfaser geführt, wobei die Lichtleitfaser endständig mit dem Katheterschlauch die Strahlung in die expandierbare Verschlußkappe abgibt.

Dabei hat die Verschlußkappe bevorzugt ein Füllvolumen von typischerweise 0,5 bis 2 cm³ - je nach Ausführungsart - und weist im entfalteten Zustand eine Länge von typischerweise 5 bis 20 mm auf, die sich ebenfalls am beabsichtigten Anwendungsgebiet orientiert. Entsprechend kann der Durchmesser im expandierten Zustand zwischen dem Durchmesser des Katheterschlauches und etwa 1 cm variieren. Die Verschlußkappe selbst ist dabei aus einem formtreuen, nur bedingt elastischen Kunststoffmaterial gefertigt, um im expandierten Zustand eine radialsymmetrische Konfiguration anzunehmen. Der Expansionszustand der Verschlußkappe wird über eine Drucksteuerung an der Einlaßseite der Kühl- und Streuflüssigkeit geregelt. Durch die Möglichkeit der Expansion des distalen Endbereichs wird der gekühlte Nahbereich vergrößert und damit der Gefahr einer lokalen Überhitzung zusätzlich entgegengewirkt. Bei ergänzender Verwendung eines Kühlthermostaten im Zuflußkreislauf der Streuflüssigkeit kann über einen Temperatursensor im Zu- und Ablauf auch eine zusätzliche Kühlwirkung an der Wandung der Verschlußkappe zum Gewebe erreicht werden, wodurch eine zusätzliche Schonung der randständigen Gewebeschichten bei der Betrahlung erreicht wird.

Bei der Anwendung eines derartigen Kathetersystems im Gewebe kann die Beaufschlagung mit einem Kompressionsdruck die Lichtausbreitung im Gewebe deutlich verbessern. Bei Anwendung eines derartigen Systems in stärker durchbluteten Körperarealen wie dem Gefäßsystem oder parenchymatösen Organen hat sich gezeigt, daß Oberflächenkühlung der expandierten Verschlußkappe durch Vorkühlung des Streumediums ein gelegentliches Anhaften von Gewebekompartimenten an der entfaltbaren Kunststoffmembran der Verschlußkappe nicht sicher verhindern kann. Dieses Problem wird erfindungsgemäß dadurch gelöst, daß die Membran der Verschlußkappe in Abstrahlrichtung, mehrere präformierte Poren erhält, durch die ein Teil der Perfusionsflüssigkeit austreten kann und so immer einen Flüssigkeitsfilm zwischen Kunststoffmembran und Gewebe aufrechterhält. Es ist daher günstig, wie oben erwähnt, biokomapatible Streuflüsigkeiten, wie beispielsweise eine Fett/Öl-Wasser-Emulsion-Infusionslösung, zu verwenden.

Bei kleinen Durchmessern des Arbeitsschaftes im Bereich von 2 mm und weniger verhindert die durch die Kühlung der Streuflüssigkeit bedingte Viskositätserhöhung eine suffiziente Spülleistung und damit Kühlleistung an der Spitze des Arbeitsschaftes.

Das zugrundeliegende Problem ist bei einer anderen vorteilhaften Weiterbildung der Erfindung zusätzlich dadurch gelöst, daß als Kühlflüssigkeit eine niedrigsiedende Flüssigkeit bzw. ein beim niedrigen Druck verflüssigbares Gas benutzt wird dergestalt, daß diese Flüssigkeit entweder koaxial oder periaxial zur laserlichtführenden Faser in den optischen Arbeitsschaft eingebracht wird, und daß die thermodynamischen Kennzahlen der Flüssigkeiten so gewählt werden, daß die beim Austreten aus der zuführenden Kapillare spontan als Aerosol verdampft und durch diesen Verdampfungsprozeß bereits Verdampfungswärme am distalen Ende des optischen Arbeitsstabes der Umgebung entzieht und dadurch ein Kühleffekt eintritt. Bei gleichzeitiger Aktivierung der Laserstrahlung wird dann auch eine vollständige Verdampfung des Aerosols erreicht, was in einem weiteren Kühleffekt resultiert. Zusätzlich wird erreicht, daß dadurch, daß sich die an der Entspannungsstelle der Kapillare austretende Flüssigkeit am kritischen Punkt befindet, sogenannte kritische Opaleszenz bzw. Vielfachstreuung an den Aerosol-Tropfen stattfindet und dadurch die gewünschte zusätzliche Streuwirkung entsteht. Durch diesen stufenweisen Verdampfungsprozeß Flüssigkeit, Aerosol, Gas wird der Umgebung sehr effizient Wärme entzogen.

Gleichzeitig hat eine derart niedrigsiedende Flüssigkeit bzw. verflüssigtes Gas die Eigenschaft, auch bei niedrigen Temperaturen ggf. unter auch unterhalb des Nullpunktes geringe Viskositäten aufzuweisen. Die Rückführung hat darüberhinaus den Vorteil, lediglich in Gasform stattzufinden, so daß Strömungswiderstände auch bei kleinen Abmessungen eines optischen Arbeitsschaftes nahezu vollständig vermieden werden bei gleichzeitig erhöhter Kühlungseffizienz.

Bei Verzicht auf Poren am distalen Ende des optischen Arbeitsschaftes kann ein derartiges System im geschlossenen Kreislauf betrieben werden, so daß als ein bevorzugtes Ausführungsbeispiel beispielsweise Fluorchlorkohlenwasserstoffe, wie insbesondere Freon, verwendet werden können, was in Anbetracht der Verwendung eines geschlossenen Systems auch im Hinblick auf mögliche Umweltbelastungen nicht bedenklich ist. Aber auch jede andere niedrigsiedende Flüssigkeit wie bestimmte Alkohole oder leicht zu verflüssigende Gase, wie Äthane, Butane usw., kommen bevorzugt als Kühl- und Streumedium in Betracht.

Ein expandierbares Ende des Arbeitsschaftes ermöglicht zusätzlich zu der photo-thermischen Behandlung die Aufbringung von Druck, insbesondere bei Gefäßapplikationen. Durch die Expansion des Gewebes wird dessen Transparenz erhöht, so daß der erreichbare Behandlungsraum vergrößert wird. Durch das Vorsehen von Poren kann Flüssigkeit zwischen die Frontfläche des distalen Endes des Arbeitsschaftes und das benachbarte Gewebe gelangen, so daß dort lokale Überhitzungen vermieden sind.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figuren 1a bis c drei vorteilhafte Ausführungsformen des erfindungsgemäßen Streulichtschaftes zur Verwendung mit einem Endoskop mit zylindrisch verrundetem, kegeligem bzw. einseitig verspiegeltem Streudom,
Figur 2 eine Vorrichtung zur Umwälzung des kühlendenden und lichtstreuenden Fluids im Blockschaltbild,
Figur 3a eine weitere Ausführungsform der Erfindung mit einem flexiblen Optikschaft,
Figur 3b ein Vorrichtung entsprechend Figur 2 zur Anwendung mit der Ausführungsform des Streulichtschafts gemäß Figur 3a,
Figur 4a eine weitere Ausführung des erfindungsgemäßen optischen Arbeitsschaftes, mit einer verdampfenden Flüssigkeit als kühlendes und streuendes Fluid,
Figur 4b eine vereinfachte Variante der Ausführung gemäß Figur 4a,
Figur 5a das Prinzipbild einer ersten Ausführungsform einer externen Verflüssigungseinrichtung zur Verwendung mit einem optischen Arbeitsschaft gemäß den Figuren 4a und 4b im Blockschaltbild sowie
Figur 5b eine Variante des Ausführungsbeispiels gemäß Figur 5a.

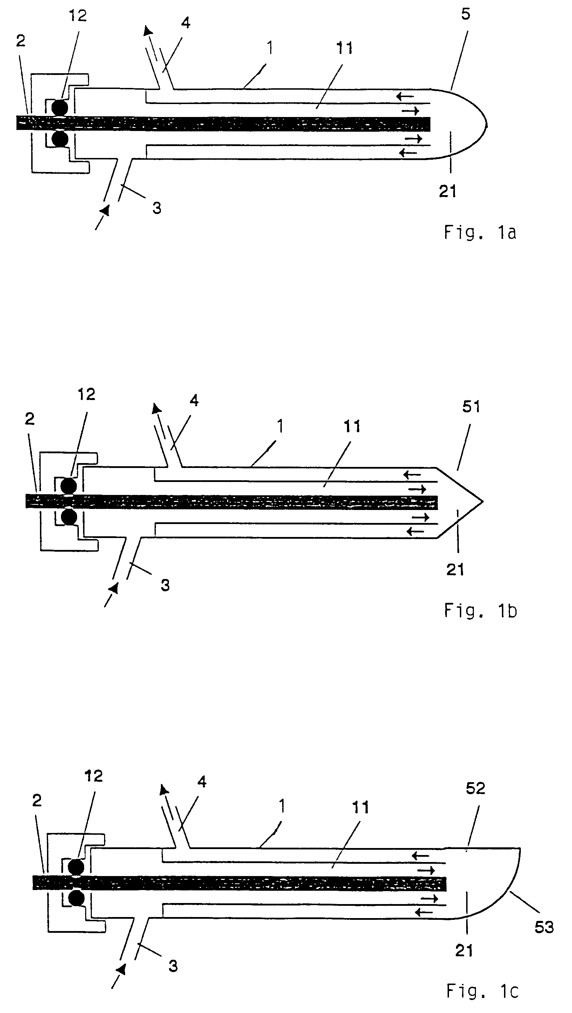

Der in den Figuren 1a bis c dargestellte starre Optikschaft 1 kann in dieser Form zur Einführung in den Arbeitskanal eines Endoskops verwendet werden. Der Optikschaft 1 trägt am distalen Ende einen lichtdurchlässigen Streulichtdom 5, in den der Lichtwellenleiter 2 über eine Einführungsschleuse 12 geführt ist. Im Inneren des Optikschaftes kann eine, das streuende und kühlende Fluid bildende, Streuflüssigkeit 21, die über den Zulauf 3 in Pfeilrichtung eingeführt werden und in einem koaxialen Hüllrohr 11 zum Abfluß 4 - ebenfalls in Pfeilrichtung - rückströmend zirkulieren. In verschiedenen Ausführungsvarianten kann dabei der Streulichtdom 5 zylindrisch gerundet (Figur 1a), kegelig (51 in Figur 1b) bzw. mit einer durch Umlenkung durch ein Fenster 52 erzeugten seitlichen Abstrahlrichtung (Figur 1c) versehen sein. Bei seitlicher Abstrahlung ist ein Spiegelelement 53 zur gerichteten Reflexion der Wirkstrahlung vorgesehen. Es ist ersichtlich, daß das streuende und kühlende Fluid von einem Bereich des proximalen Endes her zum distalen Ende geführt wird, wo es über den Lichtwellenleiter 2 mit der Strahlung beaufschlagt wird. Das Fluid wirkt streuend in der Weise, daß die am Austrittsende des Lichtwellenleiters zunächst nur gerichtet zur Verfügung stehende Strahlung nach der Streuung durch optische Mittel - wie dargestellt - zur isotropen Abstrahlung in unterschiedlichste Richtungen gelenkt werden kann, wobei die Ablenk- bzw. Richtungsauswahlmittel in einfachster Weise ausgestaltet sein können. Im Fall der Ausführung gemäß Figur 1c kann eine seitliche Raumrichtungsauswahl beispielsweise durch einfaches Drehen des Arbeitsschafts mitsamt dem Spiegel 53 vom proximalen Ende her erfolgen. Die numerische Apertur des Lichtwellenleiters ist bevorzugt größer als 0,4. Insbesondere ist er - bei einer in den Zeichnungen nicht näher dargestellten Ausführung - als Hohleiter ausgeführt, so daß das kühlende Fluid in seinem Inneren in der einen Richtung und zwischen Lichtwellenleiter und Außenwandung in der anderen Richtung fortgeleitet werden kann, so daß keine weitere Trennwand erforderlich ist.

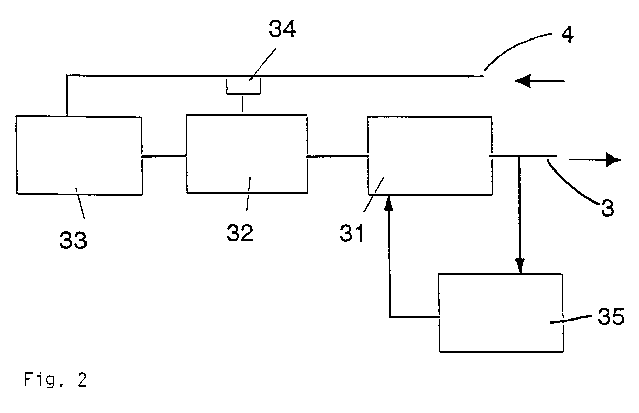

Figur 2 zeigt eine Vorrichtung zur Umwälzung der kühlenden Streulichtflüssigkeit, die in einem Ultraschallmischer und Reservoir 33 homogenisiert wird, sodann über einen Thermostat 32, der über einen Temperaturfühler in der Rücklaufleitung 34 geregelt und einer Pumpe 31 zugeführt wird. Der Sekundärdruck der Pumpe ist über einen Druckwächter 35 in der Zulaufleitung 3 regelbar.

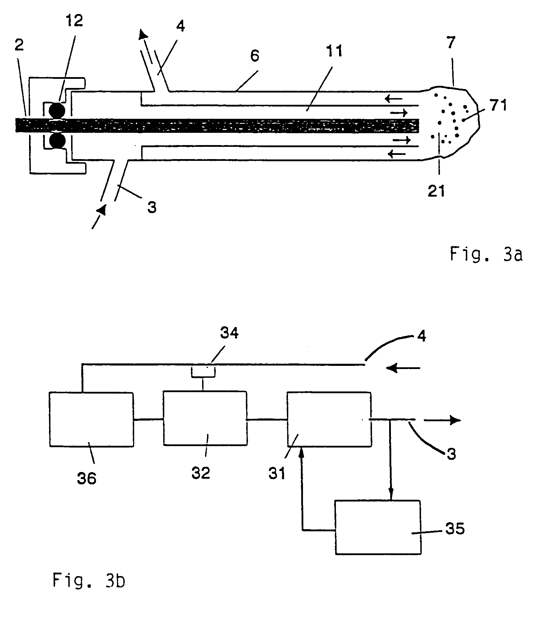

In Figur 3a ist eine weitere bevorzugte Ausführungsform der Erfindung in Form eines flexiblen Optikschaftes als Streulichtkatheter gezeigt. Dabei ist ein flexibler Katheterschlauch 6 vorgesehen, der am distalen Ende mit einer optisch transparenten, begrenzt elastischen, formstabilen Membran 7 abgeschlossen ist, in der präformierte Poren 71 eingebracht sind. Der Lichtwellenleiter 2 ist wiederum über eine Schleuse 12 in den Optikschaft eingeführt. Die kühlende Streuflüssigkeit 21 wird über einen Zulauf 3 in den mindestens zweilumigen Katheter eingebracht und strömt über den Abfluß 4 den Versorgungsgeräten zu.

Die zugehörigen Versorgungsgeräte sind in der Figur 3b im einzelnen dargestellt. Dabei wird der Abfluß 4 zunächst einem Reservoir 36 zugeführt, von dort über ein Thermostaten 32, der über einen Temperaturfühler 34 in der Rücklaufleitung gesteuert wird einer Druckpumpe 31 zugeführt, die ihrerseits über einen Druckwächter 35 im Zulauf 3 gesteuert wird.

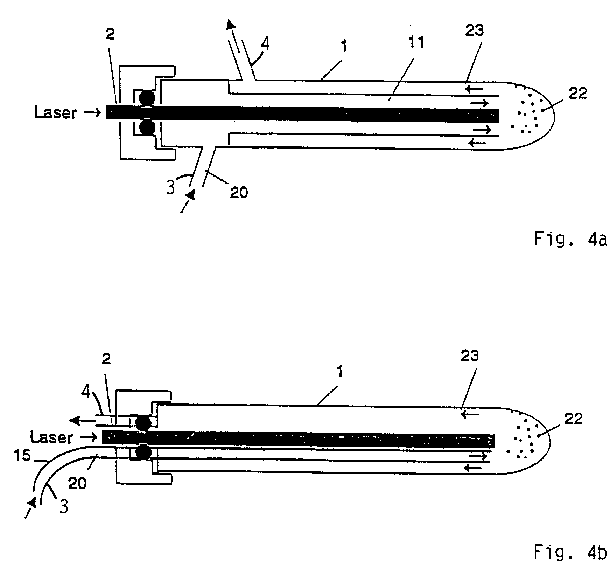

Figur 4a zeigt eine weitere Anordnung des optischen Arbeitsschaftes, in dem die zu verdampfende Flüssigkeit koaxial zur laserlichtübertragenden Faseroptik 2 in einem koaxialen Hüllkörper 11 an das distale Ende des optischen Arbeitsschaftes 1 eingebracht wird und aufgrund der am distalen Ende vorhandenen Umgebungs- bzw. stahlungsinduzierten Temperatur zunächst als Aerosol 22 verdampft wird. Das Aerosol 22 verdampft während des Applikationsprozesses vollständig zum Gas 23, das über einen Ausgangsstutzen A einem Kondensor zugeführt wird und als Flüssigkeit über den Einführstutzen E wieder dem Prozeß zugeleitet wird.

Figur 4b zeigt eine im Vergleich zu der Ausführung gemäß Figur 4a vereinfachte Anordnung, in der die Verdampferflüssigkeit 20 über ein dünnes Schlauchelement 15 an das distale Ende des optischen Arbeitsschaftes 1 vorgebracht wird. Dort findet wiederum die Aerosol- und Gasbildung statt, wobei das entstehende Gas 23 dann wiederum über einen Ausgangsstutzen einer externen Verflüssigungseinrichtung zugeführt wird.

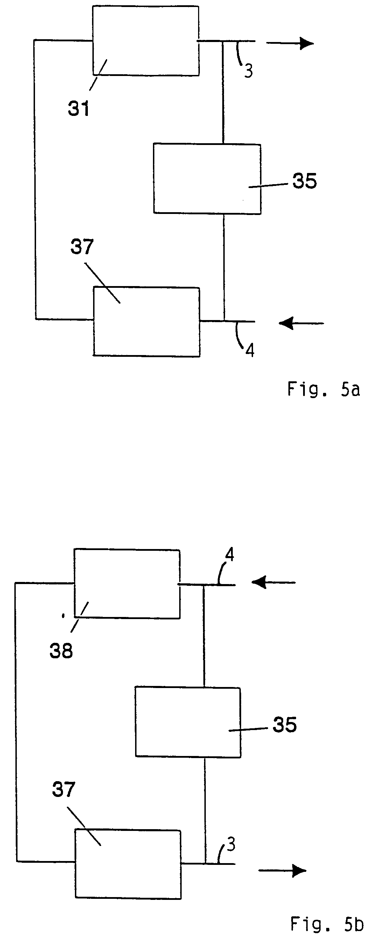

Figur 5a zeigt das Prinzipbild einer externen Verflüssigungseinrichtung für den Fall einer niedrigsiedenden Flüssigkeit, die entweder bereits bei Zimmertemperatur oder mit zusätzlicher Kühlung in einem Kondensor 37 kondensierbar ist und dann als Flüssigkeit wiederum über eine Pumpe 31 dem Prozeß zugeführt wird. Der Arbeitspunkt wird über einen Druckwächter 35 kontrolliert und eingestellt.

Figur 5b zeigt ein weiteres Ausführungsbeispiel der erfindungsgemäßen Anordnung für den Fall, daß als Streu- und Kühlmedium ein unter Druck verflüssigtes Gas verwendet wird. Dabei wird zunächst über einen Kompressor 38 das Gas komprimiert und sodann in einem Kondensor 37 verflüssigt. Der Arbeitspunkt wird wiederum über einen Druckwächter eingestellt.

In Weiterführung des Erfindungsgedankens wird der Laserstrahlung führende Lichtwellenleiter 2 am distalen Ende sich stufenförmig - oder in sonstiger Weise - verjüngend mit matter Oberfläche ausgeführt. Dies kann vorzugsweise durch Eintauchen des vom Schutzüberzug Coating befreiten Lichwellenleiters in eine Ätzflüssigkeit und anschließendes schrittweises Herausziehen erreicht werden. Als Ergebnis wird am Ende des Lichtwellenleiters ein abgestufter Konus mit mattierter Oberfläche erzeugt.

Ersichtlicherweise trägt damit das streuende Fluid zu einer ungerichteten Verteilung der Strahlung bei, wobei auch hierzu auch die Mattierung der Oberfläche des am distalen Ende gebildeten lichtdurchlässigen Doms beiträgt. Dessen geometrische Gestaltung bildet jedoch die Voraussetzung für das Erreichen einer gewünschten Intensitätsverteilung der Strahlung im Applikationsbereich.

Die intrakorporale Benutzung eines erfindungsgemäßen starren oder flexiblen Optikschaftes kann entweder wiederum über den eingangs erwähnten Arbeitsschaft eines Endoskopes erfolgen bzw. bei nichtendoskopischem Vorgehen mit einem flexiblen Katheter mittels eines Trokars oder einer üblichen Schleuse, wobei bei Anwendungen im Solid-Gewebe zunächst der Weg zum Applikationsort mit einem Mandrin aufbougiert werden muß. Anschließend wird anstelle des Mandrins der Lichtstreukatheter eingeführt und anschließend das Einführungsbesteck so weit zurückgezogen, daß es nicht mehr im Wirkbereich der Strahlung liegt.

## Patentansprüche

1. Optischer Arbeitsschaft, bei dem optische Strahlung höherer Leistung durch das distale Ende abstrahlbar ist, insbesondere zum Einführen in den Arbeitskanal eines Endoskops oder eines sonstigen Katheters, wobei für den Transport der Strahlung bis zum distalen Ende im wesentlichen ein optischer Wellenleiter (**2**) vorgesehen ist und wobei die Strahlung mindestens im distalen Bereich innerhalb eines Fluids (**21**) verläuft, das ein dispergierendes Medium ist, das den optischen Strahl beim Durchgang durch das Fluid (**21**) durch einen am distalen Ende befindlichen optischen Dom (**5**) hindurch streut, wobei der optische Dom (**5**) transparent für die optische Strahlung ist,
**dadurch gekennzeichnet,**
daß das dispergierende Fluid (**21**) im distalen Endbereich gleichzeitig eine Kühlflüssigkeit ist, die Verlustwarme abführt, die durch Streuung in der Nachbarschaft des distalen Endbereichs entsteht, und daß der Dom (**5**) aus einem opalisierenden Mineral oder opalisierenden Glas besteht.

2. Arbeitsschaft nach Anspruch 1, **dadurch gekennzeichnet**, daß Mittel zur unterschiedlichen Konzentration der Strahlung, entsprechend einer gewünschten Abstrahlcharakteristik, in Bezug auf die das distale Ende umgebenden Raumsegmente vorgesehen sind, welche insbesondere in der geometrischen Gestaltung (**51**) der Form des optischen Doms (**5**) bestehen.

3. Arbeitsschaft nach Anspruch 2, **dadurch gekennzeichnet,** daß die Mittel zur unterschiedlichen Konzentration der Strahlung vom distalen Ende her bezüglich der Raumrichtung steuerbar sind.

4. Arbeitsschaft nach Anspruch 3, **dadurch gekennzeichnet,** daß im Bereich des distalen Endes mindestens ein einstellbares Spiegelsegment (**53**) vorgesehen ist.

5. Arbeitsschaft nach einem der vorangehenden Ansprüche, **dadurch gekennzeichnet,** daß das Fluid (**21**) einen Phasenstreuer bildet.

6. Arbeitsschaft nach einem der vorangehenden Ansprüche, **dadurch gekennzeichnet,** daß Mittel zur Förderung des Kühlfluids koaxial zum Strahlung führenden Wellenleiter in Richtung auf das distale Ende vorgesehen sind.

7. Arbeitsschaft nach einem der vorangehenden Ansprüche, **dadurch gekennzeichnet,** daß zur Kühlung und als optisch streuendes Fluid (**21**) eine Emulsion aus Öl, insbesondere Silikonöl, und Wasser und/oder als biokompatible Streuflüssigkeit eine Fett/Öl-Wasser-Emulsion-Infusionslösung vorgesehen ist.

8. Arbeitsschaft nach Anspruch 7, **dadurch gekennzeichnet,** daß ein Ultraschallschwinger zum Mischen bzw. In-Mischung-halten der Emulsion aus Öl und Wasser vorgesehen ist.

9. Arbeitsschaft nach Anspruch 8, **dadurch gekennzeichnet,** daß zur Stabilisierung der mittels Ultraschall erzeugten Emulsion eine Detergenz vorgesehen ist.

10. Arbeitsschaft nach einem der vorangehenden Ansprüche, **dadurch gekennzeichnet,** daß als Fluid (**21**) eine leicht verdampfbare Flüssigkeit vorgesehen ist.

11. Arbeitsschaft nach Anspruch 10, **dadurch gekennzeichnet,** daß es sich bei der verdampfbaren Flüssigkeit um Alkohol und/oder Fluorkohlenwasserstoff handelt.

12. Arbeitsschaft nach einem der Ansprüche 10 oder 11, **dadurch gekennzeichnet,** daß die verdampfbare Flüssigkeit durch eine Kondensator-Pumpen bzw. Kompressor-Kondensator-Anordnung rekondensier- und als Flüssigkeit wiederum dem Prozeß zuführbar ist.

13. Arbeitsschaft nach einem der Ansprüche 10 bis 12, **dadurch gekennzeichnet,** daß ein getrennter Schlauch bzw. ein Rohr zur Zuführung der verdampfbaren Flüssigkeit in die distale Verdampfungsregion vorgesehen ist.

14. Arbeitsschaft nach einem der vorangehenden Ansprüche, **dadurch gekennzeichnet,** daß ein flexibler Katheter (**6**) als Führungsschaft für den optischen Lichtwellenleiter (**2**) zur Übertragung der Wirkstrahlung vorgesehen ist.

15. Arbeitsschaft nach einem der vorangehenden Ansprüche, **dadurch gekennzeichnet,** daß die Temperatur des kühlenden und optisch streuenden Fluids (**21**) regelbar ist.

16. Arbeitsschaft nach Anspruch 14, **dadurch gekennzeichnet,** daß der Katheter (**6**) an seinem distalen Ende mit einer optisch transparenten expandierbaren Verschlußkappe (**7**) versehen ist.

17. Arbeitsschaft nach Anspruch 16, **gekennzeichnet durch** eine Drucksteuerung zur kontrollierten Expansion der Verschlußkappe (**7**).

18. Arbeitsschaft nach einem der Ansprüche 16 oder 17, **dadurch gekennzeichnet,** daß als Material für die Verschlußkappe (**7**) eine formstabile, beschränkt elastische Kunststoffmembran vorgesehen ist.

19. Arbeitsschaft nach einem der Anspruch 18, **dadurch gekennzeichnet,** daß die Kunststoffmembran der Verschlußkappe (**7**) präformierte Poren zum Flüssigkeitsaustritt aufweist.

20. Arbeitsschaft nach einem der vorangehenden Ansprüche, **dadurch gekennzeichnet,** daß die Strahlung durch Laserstrahlung gebildet wird.

21. Arbeitsschaft nach einem der vorangehenden Ansprüche, **dadurch gekennzeichnet,** daß der Wellenlängenbereich im wesentlichen 400 bis 1400 nm beträgt.

22. Arbeitsschaft nach einem der vorangehenden Ansprüche, **dadurch gekennzeichnet,** daß das distale Ende des Lichtwellenleiters gestuft oder in sonstiger Weise sich zu seinem Ende hin verjüngend ausgebildet ist.

23. Arbeitsschaft nach einem der vorangehenden Ansprüche, **dadurch gekennzeichnet,** daß der Lichtwellenleiter eine numerische Apertur von größer als 0,4 aufweist.

24. Arbeitsschaft nach einem der vorangehenden Ansprüche, **dadurch gekennzeichnet**, daß der Lichtwellenleiter als Hohlleiter ausgebildet ist.

## Claims

1. An optical working shaft wherein optical beams of increased output can be radiated through the distal end, in particular for insertion into the working tube of an endoscope or other catheter, wherein an optical waveguide (2) conveys the beam essentially to the distal end, and wherein at least in the distal area the path of the beam to the distal end extends in a fluid (21), which is a radiation dispersing medium and which scatters the optical beam through an optical dome (5) at its distal end as the beam traverses the fluid (21), wherein the optical dome (5) is transparent to the optical beam
characterised in that
in the distal area the dispersing fluid (21) is simultaneously a coolant which removes waste heat generated within the distal area and the dome (5) is composed of an opalescent mineral or opalescent glass.

2. A working shaft in accordance with claim 1, characterised in that means for the different concentration of the beam, corresponding to a desired beam characteristic, are provided in relation to the spatial segments surrounding the distal end, which consist in particular in geometric design (51) of the shape of the optical dome (5).

3. A working shaft in accordance with claim 2, characterised in that the means for the different concentration of the beam from the direction of the distal end are controllable in respect to the spatial direction.

4. A working shaft in accordance with claim 3, characterised in that at least one adjustable mirror element (53) is provided in the area of the distal end.

5. A working shaft in accordance with one of the preceding claims, characterised in that the fluid (21) forms a phase scatterer.

6. A working shaft in accordance with one of the preceding claims, characterised in that means are provided for conveying the cooling fluid in the direction toward the distal end coaxially with the waveguide guiding the beam.

7. A working shaft in accordance with one of the preceding claims, characterised in that an emulsion of oil (in particular silicon oil) and water is provided for cooling and as optically scattering fluid (21) and/or a fat/oil-water-emulsion infusion solution as a bio-compatible scattering fluid.

8. A working shaft in accordance with claim 7, characterised in that an ultrasonic oscillator is provided for mixing or keeping the emulsion of oil and water in suspension.

9. A working shaft in accordance with claim 8, characterised in that a dispersion is provided for stabilising the emulsion created by means of ultrasound.

10. A working shaft in accordance with one of the preceding claims, characterised in that an easily vaporising fluid is provided as the fluid (21).

11. A working shaft in accordance with claim 10, characterised in that the vaporisable fluid is alcohol and/or fluorocarbon.

12. A working shaft in accordance with one of claims 10 or 11, characterised in that the vaporisable fluid can be recondensed by means of a condenser-pump or compressor-condenser arrangement and can be again supplied to the process as a fluid.

13. A working shaft in accordance with one of claims 10 to 12, characterised in that a separate hose or a tube is provided for supplying the vaporisable fluid to the distal vaporisation region.

14. A working shaft in accordance with one of the preceding claims, characterised in that a flexible catheter (6) is provided as the guide shaft for the optical waveguide (2) for transmitting the active beam.

15. A working shaft in accordance with one of the preceding claims, characterised in that the temperature of the cooling and optically scattering fluid (21) is controllable.

16. A working shaft in accordance with claim 14, characterised in that at its distal end the catheter is provided with an optically transparent expandable closure cap (7).

17. A working shaft in accordance with claim 16, characterised by a pressure control for the controlled expansion of the closure cap (7).

18. A working shaft in accordance with one of claims 16 or 17, characterised in that a dimensionally stable, partially elastic plastic diaphragm is provided as the material for the closure cap (7).

19. A working shaft in accordance with claims 16 or 18, characterised in that the plastic diaphragm of the closure cap (7) has preformed pores for the exit of fluid.

20. A working shaft in accordance with one of the preceding claims, characterised in that the beam is provided by a laser beam.

21. A working shaft in accordance with one of the preceding claims, characterised in that the wavelength range is essentially 400 to 1400 nm.

22. A working shaft in accordance with one of the preceding claims, characterised in that the distal end of the optical waveguide is embodied to be stepped or to taper toward its end in another way.

23. A working shaft in accordance with one of the preceding claims, characterised in that the optical waveguide has a numerical aperture of greater than 0.4.

24. A working shaft in accordance with one of the preceding claims, characterised in that the optical waveguide is embodied as a hollow waveguide.

## Revendications

1. Une tige de travail où un rayonnement optique de plus forte puissance peut être émis au travers de l'extrémité distale, en particulier pour l'insertion dans le canal de travail d'un endoscope ou d'un autre cathéter où un guide d'ondes optique (2) transporte la radiation essentiel dans la zone distale et où au moins le chemin du faisceau jusqu'au l'extrémité distale est prolonge à l'intérieur d'un fluide (21), étant une médium dispersant, que diffuse la radiation à travers un dôme optique (5) au fin distale qui est transparent pour la radiation
caractérisé par le fait que
dans la zone distale le fluide (21) assure une fonction de réfrigération pour l'élimination des calories excédentaires générées par la diffusion dans la proximité de la zone de l'extrémité et que le dôme (5) est constitue d'un minéral opalescent ou d'un verre opalescent.

2. Une tige de travail conformément à la revendication 1, caractérisée par le fait que des moyens, pour les différentes concentrations de faisceaux correspondant à des caractéristiques souhaitées, sont assurés en fonction des segments spatiaux entourant l'extrémité distale, qui dépend en particulier du concept géométrique (51) de la forme du dôme optique (5).

3. Une tige de travail conformément à la revendication 2, caractérisée par le fait que les moyens pour les différentes concentrations du faisceau issu de l' extrémité distale sont contrôlables quant à la direction spatiale.

4. Une tige de travail conformément à la revendication 3 caractérisée par le fait qu'au moins un élément miroir ajustable (53) est disponible dans la zone de l'extrémité distale.

5. Une tige de travail conformément à une des revendications précédentes caractérisée par le fait que le fluide (21) forme un diffuseur de phase.

6. Une tige de travail, conformément à une des revendications précédentes caractérisée par le fait que des moyens sont assurées pour conduire le liquide de refroidissement vers l'extrémité distale coaxialement au guide d'onde optique transportant la radiation.

7. Une tige de travail conformément à une des revendications précédentes caractérisée par le fait soit une émulsion d'huile (en particulier huile de silicone) et d'eau, soit une solution infusion - émulsion corps gras/huile - eau agisse comme fluide (21) de refroidissement et de diffusion optique.

8. Une tige de travail conformément à la revendication 7 caractérisée par le fait qu'un oscillateur ultra-sonique assure le mélange de l'émulsion huile - eau et le garde en suspension.

9. Une tige de travail, conformément à la revendication 8 caractérisée par le fait qu'une dispersion est assurée pour la stabilisation de l'émulsion créée au moyen d'ultrasons.

10. Une tige de travail conformément à une des revendications précédentes caractérisée par le fait qu'un liquide facilement vaporisable soit à la base du liquide de refroidissement.

11. Une tige de travail, conformément à la revendication 10, caractérisée par le fait que le liquide vaporisable est de l'alcool et/ou un fluorocarbone.

12. Une tige de travail conformément aux revendications 10 ou 11, caractérisée par le fait que le liquide vaporisable peut être re-condensé au moyen d'une pompe-condenseur ou d'un système compresseur/condenseur et peut ensuite être recyclée dans le procédé comme fluide.

13. Une tige de travail conformément aux revendications 10 à 12, caractérisée par le fait qu'un tuyau flexible ou un tube puissent assurer le transport du fluide (21) vaporisable vers la zone de vaporisation distale.

14. Une tige de travail conformément à une des revendications précédentes, caractérisée par le fait qu'un cathéter flexible (6) puisse servir de tube de guidage à la fibre optique (2) devant transmettre le faisceau actif.

15. Une tige de travail, conformément à une des revendications précédentes caractérisée par le fait que la température du liquide (21) de refroidissement et de diffusion optique soit contrôlable.

16. Une tige de travail conformément à la revendication 14, caractérisée par le fait que l'extrémité distale du cathéter soit équipée d'un capuchon de fermeture optiquement transparente et dilatable.

17. Une tige de travail conformément à la revendication 16, caractérisée par un contrôle de pression permettant de contrôler la dilatation du capuchon (7)de fermeture.

18. Une tige de travail, conformément à une des revendications 16 ou 17, caractérisée par le fait qu'un diaphragme plastique partiellement élastique et stable dimensionnellement soit la base du matériau du capuchon de fermeture.

19. Une tige de travail, conformément à une des revendications 16 ou 18, caractérisée par le fait que le diaphragme plastique du capuchon (7) dispose de pores préformées servant à l'évacuation du fluide (21).

20. Une tige de travail conformément à une des revendications précédentes caractérisée par le fait que le faisceau est assuré par une faisceau laser.

21. Une tige de travail conformément à une des revendications précédentes, caractérisée par le fait que la plage de longueurs d'onde est essentiellement 400 à 1400 nm.

22. Une tige de travail conformément à une des revendications précédentes, caractérisée par le fait que l'extrémité distale de la fibre soit être échelonnée ou taillée vers son extrémité.

23. Une tige de travail, conformément à une des revendications précédentes caractérisée par le fait que la fibre optique ait une ouverture numérique supérieure à 0,4

24. Une tige de travail, conformément à une des revendications précédentes, caractérisée par le fait que la fibre optique est conçue comme un guide d'onde creux.
